# EUROPEAN PATENT SPECIFICATION

(11) **EP 1 919 381 B1**
(45) Date of publication and mention of the grant of the patent: **16.04.2014**
(21) Application number: 06762808.1
(22) Date of filing: 25.07.2006
(51) Int. Cl.: A61B 17/64, A61B 17/17

(54) **APPARATUS FOR EXTERNAL FIXATION OF THE PELVIC RING**
GERÄT ZUR EXTERNEN FIXIERUNG DES BECKENRINGS
APPAREIL DESTINÉ À UNE FIXATION EXTERNE À L ANNEAU PELVIEN

(30) Priority: 28.07.2005 IT PD20050232
(43) Date of publication of application: 14.05.2008
(73) Proprietor: Mikai S.P.A., 16145 Genova (IT)
(72) Inventor: MASSE' Alessandro, I-10100 Torino (IT); ALOJ, Domenico, I-10100 Torino (IT); BIASIBETTI, Antonio, I-10100 Torino (IT)
(74) Representative: Forattini, Amelia
(86) International application number: PCT/EP2006/007321
(87) International publication number: WO 2007/017097

(56) References cited:
- EP-A2- 0 314 021
- RU-C2- 2 195 896
- SU-A1- 1 149 960
- US-A- 5 443 465
- US-A- 5 601 550
- US-A- 5 630 814
- US-A- 6 162 222
- GREEN S A: "COMPONENTS OF THE ILIZAROV SYSTEM" TECHNIQUES IN ORTHOPAEDICS, GAITHERSBURG, MD, US, vol. 5, no. 4, December 1990 (1990-12), pages 1-11, XP000667188

## Description

The present invention relates to an apparatus for external fixation of the pelvic ring, for the surgical and orthopedic treatment of fractures of the human pelvis.

Conventional apparatuses for external fixation of the pelvic ring, used in the current practice of surgical orthopedic procedures suitable to achieve reduction of pelvic fractures, generally comprise left and right grip means that the surgeon inserts and engages by screwing in insertion/engagement holes provided in the left and right iliac bone. Those apparatuses also include left and right locking means, that respectively lock the left and right grip means together. Those conventional apparatuses are provided with left and right articulation means for articulating the left and right locking means. Those conventional apparatuses include joining means, which are required in order to provide the transabdominal joining of the left and right articulation means.

The procedure for applying those apparatuses, during orthopedic surgical procedures for reducing pelvic fractures, generally comprises the following steps.

In a first preliminary step, the orthopedic surgeon determines the positions on the left and right iliac bone where the insertion/engagement holes for the grip means are to be provided by means of a palpatory examination of the region of application of the apparatus.

In a second preliminary step, the surgeon incises the skin of the patient, in the region of application of the apparatus, at the positions determined earlier, enough to expose the edges of the left and right iliac bone. In a third preliminary step, the orthopedic surgeon performs, by using traditional drilling means, the holes for insertion/engagement on the left and right iliac bone. In a first application step, the surgeon inserts the grip means in the insertion/engagement holes and engages them by screw coupling therein. In a second application step, the surgeon locks the left and right grip means to the left and right locking means, respectively. Then, in a third application step, the surgeon provides the connection of the left and right locking means by virtue of the articulation and joining means. At the end of this application procedure, the apparatus forms a solid structure, which temporarily replaces the pelvis until the fractures that affect it have been resolved completely.

The main drawback observed in the prior art apparatuses is that they are unlikely to ensure conditions of fixation of the fractured pelvis that are suitable to provide the abdomen containment action performed by the intact pelvis. This condition causes the onset of the risk of hemorrhages inside the abdomen of the patient, which can seriously endanger his life. This drawback arises from the operating difficulties encountered by the orthopedic surgeon in correctly performing the steps that precede application, which are the most delicate moments of the entire procedure for applying the apparatus. In particular, the preliminary steps for identifying the positions on the left and right iliac bone where the insertion/engagement holes are to be provided and for providing the insertion/engagement holes are particularly critical and depend heavily on the skill of the orthopedic surgeon. The complexity of execution of those steps can be worsened by particular situations in which the surgeon must operate. For example, performing those preliminary steps is particularly difficult in highly overweight patients and when the surgeon is forced to perform the apparatus application procedure in urgent or critically urgent conditions. This complexity of execution entails that the positions and orientation of the grip means tend to be inaccurate and inadequate to achieve, by applying the apparatus, an ideal condition of fixation of the fractured pelvis. This fact is the origin of the drawbacks described above.

US-6162222 **discloses an apparatus for the external fixation of the pelvis comprising a frame supporting two clamps for respective sets of screws. The frame has a transversely extending member which is articulable between a first position and a second position such that the transversely extending member is displaced in an inferior/superior direction when the frame is articulated from the first position to the second position.**

SU1149960 discloses an apparatus for the external fixation of the pelvis **comprising a frame constituted by a plurality of rods connected by threaded connections.**

US5630814 **discloses a fastener for wires or pins used with an external fixation device to rigidly immobilize bone fragments. The fastener comprises a fixation bolt with either a transverse asymmetrical shank bore, or a transversely slotted bolthead for providing multiple contact surfaces to secure the wires or pins, regardless of their diameters. The fastener can include a washer or a threaded washer-like member having nonparallel opposing planar surfaces with a transverse slot in one.**

US5443465 **discloses an osteosynthesis aid comprising a central body formed of two link members, each of which has an elongate slot through which a lock bolt is passed to provide a releasably clamped joint.**

EP0314021 **discloses a fixing device constituted by a tubular support bar, in which at least the inner contour is of a non-circular cross-section; holding devices adapted to the cross-sectional profile of the support bar, are guided longitudinally displaceably but rotationally fixed on the support bar. Locking screws can be fixed in any desired position of the support bar and clamping devices are arranged on the holding devices.**

The aim of the present invention is to provide an apparatus for external fixation of the pelvic ring that is capable of solving the drawbacks observed in prior art devices.

An object of the invention is to provide an apparatus that can ensure conditions of fixation of the fractured pelvis that are suitable to apply the same abdomen containment action performed by the intact pelvis.

A further object of the invention is to provide an apparatus that allows the procedure for applying the apparatus to become substantially independent of the execution skills of the orthopedic surgeon and of the operating conditions in which the orthopedic surgeon is forced to work.

A further object of the invention is to provide an apparatus that has a simplified structure with respect to prior art apparatuses, so as to contain production costs and the times required for application procedures.

A further object of the invention is to provide an apparatus that can be manufactured predominantly with radiotransparent materials, so as to facilitate the execution of X-rays and analysis of X-ray images.

This aim and these and other objects, which will become better apparent hereinafter, are achieved by an apparatus for external fixation of the pelvic ring, as claimed in the **appended claims.**

Further characteristics and advantages will become better apparent from the description of an embodiment of the invention illustrated by way of example in the accompanying drawings, wherein:
Figure 1 is a front perspective view of the apparatus according to the invention, in the condition applied to a pelvic ring;

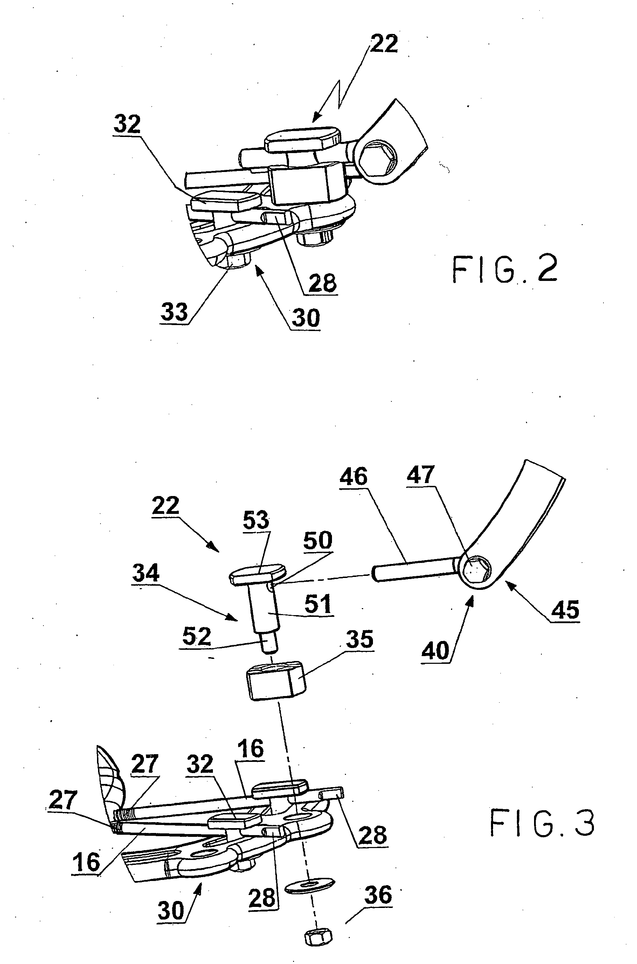

Figure 2 is a detailed perspective view of the right locking and articulation means of the apparatus;
Figure 3 is an exploded perspective view of the articulation means shown in Figure 2;

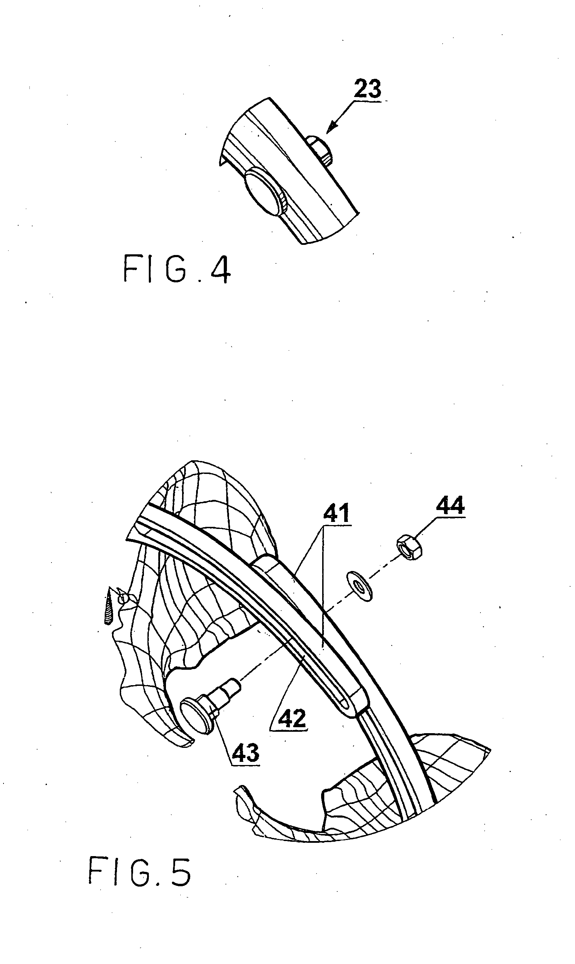

Figure 4 is a detailed perspective view of the joining means of the apparatus;
Figure 5 is an exploded perspective view of the joining means of the apparatus;

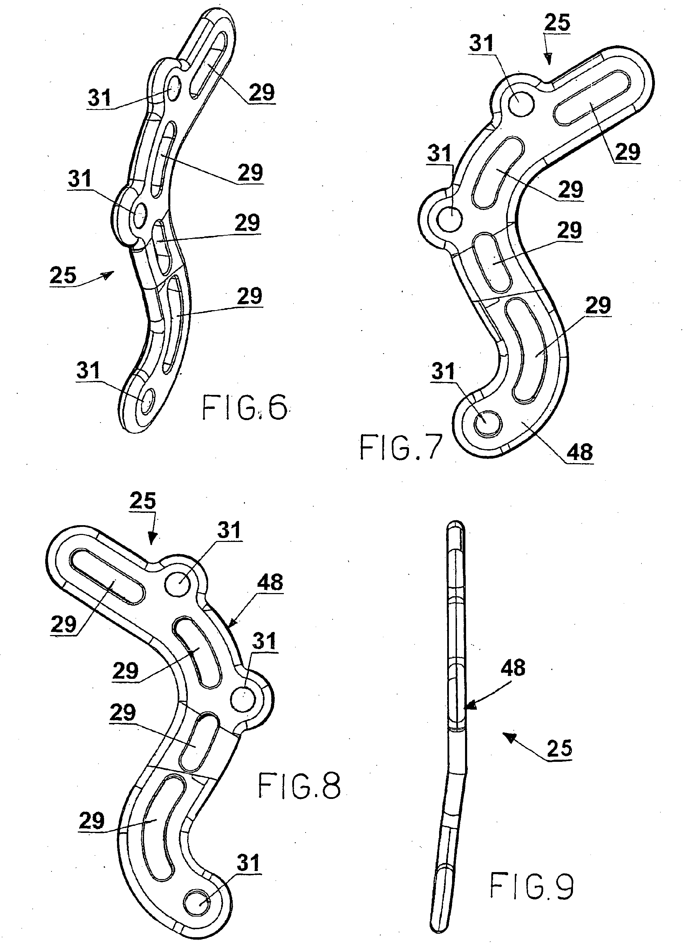

Figures 6, 7, 8 and 9 are respectively a perspective view, a front view, a rear view and a side view of the left guiding means of the apparatus;

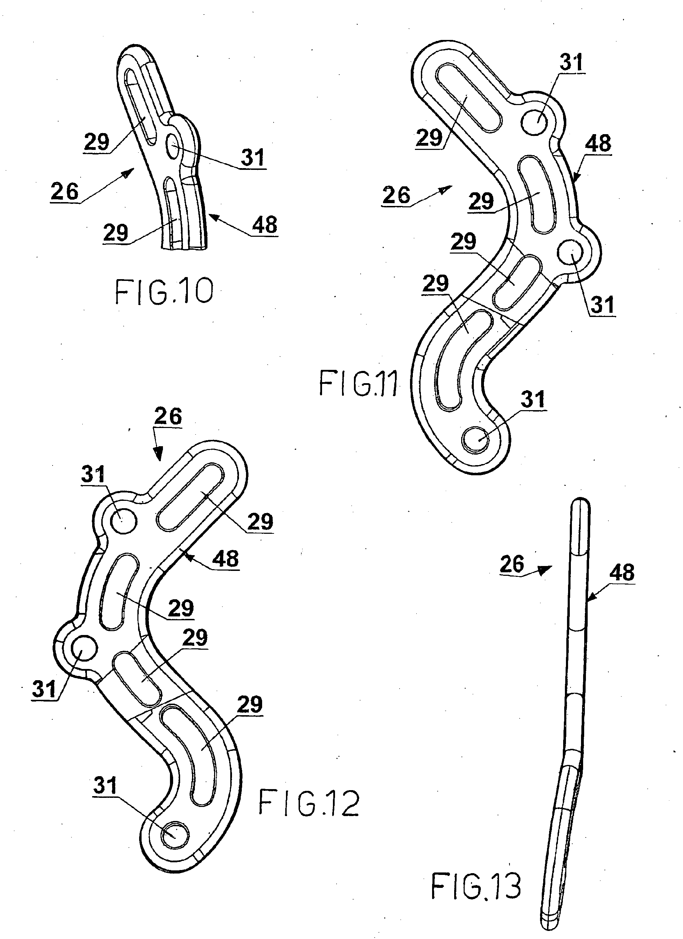

Figures 10, 11, 12 and 13 are respectively a perspective view, a front view, a rear review and a side view of the right guiding means of the apparatus.

With reference to the figures, an external fixation apparatus, generally designated by the reference numeral 13, for a pelvic ring 14, comprises left and right grip means 15, 16, which are shown in the condition in which they are engaged in the insertion/engagement holes (not shown) provided respectively in the left and right iliac bone 17, 18 of the pelvic ring 14. The apparatus 13 is provided with left and right locking means, generally designated by the reference numerals 19 and 20, which mutually lock respectively the left and right grip means 15, 16. Left and right articulation means, generally designated by the reference numerals 21 and 22, provide an articulated connection of the left and right locking means 19 and 20. Joining means, generally designated by the reference numerals 23 and 24, join the left and right articulation means 21 and 22 from one side to the other of the abdomen of the patient. Left and right guiding means, generally designated by the reference numerals 25 and 26, are provided for guiding drilling means (not shown), which the orthopedic surgeon uses in producing the insertion/engagement holes. The left and right guiding means 25, 26 allow correct execution of the insertion/engagement holes, independently of the skill of the orthopedic surgeon, as described in greater detail hereinafter.

The left and right grip means 15,16 are formed by rod members, provided at one end with a tip portion provided with an external thread 27, which is inserted and partially engaged by screwing in the insertion/engagement holes and, at the opposite end, with a squared tip portion 28 for coupling to an actuation tool (not shown). The left and right locking means 19, 20 include a series of slots 29, that are provided in the guiding means 25 and 26, fixing members 30, that fix the grip means 15, 16 to the guiding means 25, 26, and pluralities of holes 31, also provided in the guiding means 25 and 26 in which the articulation means 21 and 22 are engaged. A series of slots 29 is formed in the longitudinal central region of the guiding means 25, 26 and a plurality of holes 31 are formed along the peripheral profile of the guiding means 25, 26, which is directed toward the outside of the apparatus 13. The fixing members 30 include screws 32 for engaging the grip means 15, 16 and nuts 33 for locking the engagement screws 32, which are engaged by screw coupling on the engagement screws 32 so as to lock the engagement screws 32 to the guiding means 25, 26.

The engagement screws 32 are formed by a stem which has, at one end, a portion provided with an external thread suitable to mate with the thread provided on the locking nut 33, are provided, on the opposite side with respect to the threaded portion, with a head whose shape is suitable for mating with an actuation tool (not shown), and are provided, proximate to the head, with a transverse hole, which is formed on the stem and is crossed by a grip means 15, 16. The articulation means 21, 22 are provided substantially like the fixing members 30. The articulation means 21, 22 include articulation screws 34 in order to engage the joining means 23 and 24, articulation spacers 35 to be inserted in the articulation screws 34, and articulation nuts 36 engaged on the articulation screws 34 in order to fasten them to the guiding means 25, 26. The articulation screws 34 are formed by an articulation stem 51 which has, at one end, a threaded portion 52 which is suitable externally for mating with the thread provided on the articulation nut 36, has, on the opposite side with respect to the threaded portion, a head 53 suitable for mating with an actuation tool (not shown), and has a transverse hole 50 formed in the articulation stem proximate to the head, which partially accommodates the joining means 23, 24 namely a tab 46.

The joining means 23, 24 in turn are each constituted by two arc-like members 37 and 38, by adjustable connecting members, generally designated by the reference numerals 39, which mutually connect the joining members 37, 38, allowing to perform an adjustment, and by articulated connecting members, generally designated by the reference numeral 40, which articulately connect the joining members 37, 38 to the articulation means 21, 22. In greater detail, the adjustable connecting members 39 are provided at first ends 41 of the joining members 37, 38 and include two elongated slots 42 provided in the joining members 37 and 38, a locking screw 43 inserted in both slots 42, and finally a fixing nut 44, which is screwed onto the locking screw 43 until the joining members 37, 38 are fixed to each other. The articulated connecting members 40 are provided at second ends 45 of the joining members 37, 38 and include tabs 46, which are connected to the second ends 45 by virtue of articulation screws 47, which allow to articulate the tabs 46 with respect to the joining members 37, 38.

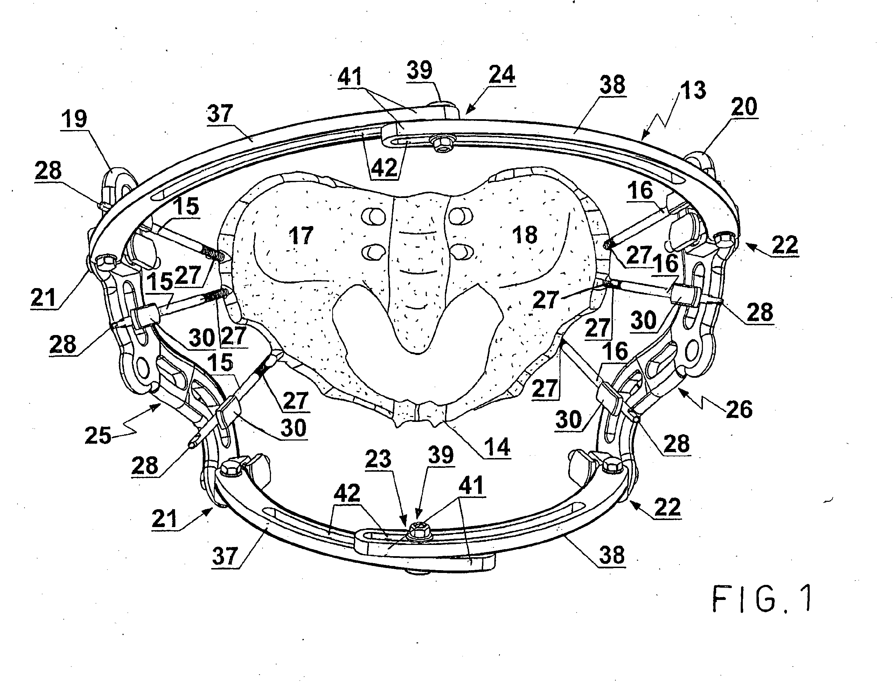

The left and right guiding means 25, 26 include supporting bodies 48, that have a substantially S-shaped curved profile. In detail, this profile substantially reproduces the peripheral shape of the iliac bones 17 and 18 approximately in the central region between the iliac crest and the pubic symphysis, as shown in Figure 1. To conclude, the locking means 19 and 20, the articulation means 21 and 22, the joining means 23 and 24 advantageously can be made of radiotransparent materials.

The procedure for applying the apparatus 13 to the pelvic ring 14 is described hereafter. As in a conventional procedural technique, the operating procedure can be divided into steps that are preliminary to the application of the apparatus and into steps of application. In a first preliminary step, the surgeon identifies the positions on the crests of the left and right iliac bone 17, 18 where the first holes for insertion/engagement of the first left and right grip means 15, 16 are to be provided, by palpatory examination of the region of application of the apparatus 13. In a second preliminary step, the surgeon incises the skin of the patient, in the region of application of the apparatus 13 at the positions determined earlier, so as to expose the edges of the crests of the left and right iliac bone 17, 18. In a third preliminary step, the surgeon performs, with the aid of traditional drilling means, the holes for insertion/engagement on the crests of the left and right iliac bone 17, 18 at the positions determined earlier. In a first application step, the surgeon inserts the first grip means 15, 16 in the insertion/engagement holes provided earlier and engages them by screwing therein. In a second application step, the surgeon temporarily fits on the first grip means 15, 16 the left and right locking means 19, 20 and the left and right guiding means 25, 26, arranging the profile of the latter in accordance with the external profile of the pelvis 14. In a third application step, the surgeon arranges the drilling means approximately tangent to the internal side of the left and right guiding means 25, 26, with the drilling tool directed toward the region of application of the apparatus 13 in successive positions so as to descend from the crests of the iliac bones 17, 18 at the series of slots 29, so as to determine precisely the additional positions where the other holes for insertion/engagement of the remaining grip means 15, 16 are to be provided. In a fourth application step, the surgeon incises the skin at the positions determined earlier, and by arranging the drilling means as described above produces the remaining insertion/engagement holes, which are mutually oriented correctly. In a fifth step of application, the surgeon inserts and screws the remaining grip means 15, 16 in the insertion/engagement holes that have just been provided and locks the grip means 15, 16 with the left and right locking means 19 and 20. In a sixth application step, the surgeon proceeds with the assembly of the apparatus 13, fixing the left and right articulation means 21, 22 to the locking means 19, 20. In a seventh step, the surgeon completes the assembly of the apparatus 13 by coupling and appropriately fixing the joining means 23, 24 to the articulation means 21, 22.

In practice it has been found that the apparatus achieves the intended aim and objects, overcoming the drawbacks observed in the prior art. The apparatus in fact allows to position and orient the grip means correctly, so as to achieve, at the end of the application of the apparatus, conditions of fixation of the fractured pelvis that are suitable to apply the same abdomen containment action performed by the intact pelvis. With the apparatus according to the present invention, providing the insertion/engagement holes of the grip means is an operation substantially independent of the skills of the surgeon and of the operating conditions. Also, the apparatus has a structure which allows to contain production costs and application times. Finally, the apparatus can be made almost entirely of radiotransparent materials, so as to facilitate both the execution of X-rays exams on the region of application of the apparatus and the analysis of the corresponding results.

## Claims

1. An apparatus (13) for external fixation of the pelvic ring (14), comprising left grip means (15) and right grip means (16) to be engaged in insertion/engagement holes formed respectively in the left (17) and right (18) iliac bone of said pelvic ring (14), left locking means (19) and right locking means (20) for mutually locking respectively said left grip means (15) and said right grip means (18), left articulation means (21) and right articulation means (22) adapted to articulately connect said left (19) and right (20) locking means, and transabdominal joining means (23, 24) for joining said left and right articulation means (21, 22); left and right guiding means (25, 26) for guiding drilling means used by the surgeon to provide said insertion/engagement holes so as to allow correct execution of said insertion/engagement holes; said apparatus being **characterized in that** said guiding means (25, 26) comprise supporting bodies (48) which have a substantially S-shaped curved profile.

2. The apparatus according to claim 1, **characterized in that** said profile substantially reproduces the peripheral shape of the iliac bones approximately in the central region comprised between the iliac crest and the pubic symphysis.

3. The apparatus according to claim 1, **characterized in that** said left and right grip means (15, 16) comprise rod members having, at one end, a tip provided with an external thread (27) adapted to be inserted and engaged by screwing at least partially in said insertion/engagement holes and, at the opposite end, a squared tip portion (28) in order to allow mating with an actuation tool.

4. The apparatus according to claim 3, **characterized in that** said left and right locking means (19, 20) comprise slots (29) formed in said guiding means (25, 26), fixing members (30) for fixing said grip means (15, 16) to said guiding means (25, 26), and guide holes (31) formed in said guiding means (25, 26) for the engagement of said articulation means (21, 22).

5. The apparatus according to claim 4, **characterized in that** said slots (29) are provided in a longitudinal central region of said guiding means (25, 26).

6. The apparatus according to claim 4, **characterized in that** said guide holes (31) are formed along a peripheral profile of said guiding means (25, 26) that is directed toward the outside of said apparatus (13).

7. The apparatus according to claim 4, **characterized in that** said fixing members (30) comprise engagement screws (32) for engaging said grip means (15, 16) and locking nuts (33) for locking said engagement screws (32) so as to lock said engagement screws (32) to said guiding means (25, 26).

8. The apparatus according to claim 7, **characterized in that** each of said engagement screws (32) comprises a stem provided, at one end, with a threaded portion having an external thread adapted to mate with said locking nut (33), and, at the opposite end with respect to said threaded portion, with a head having a shape which is suitable to mate with an actuation tool and which is provided, proximate to said head, with a transverse hole formed in said stem and crossed by one of said grip means (15, 16).

9. The apparatus according to claim 8, **characterized in that** said articulation means (21, 22) comprise articulation screws (34) for the engagement of said joining means (23, 24), articulation spacers (35) to be fitted on said articulation screws (34), and articulation nuts (36) engaged on said articulation screws (34) in order to lock said articulation screws (34) to said guiding means (25, 26).

10. The apparatus according to claim 9, **characterized in that** each of said articulation screws (34) comprises an articulation stem (51) provided, at one end, with a portion having an external thread suitable to mate with said articulation nut (36), and, at the opposite end, with a head (53) having a shape that is suitable to mate with an actuation tool and is provided, proximate to said head, with a transverse hole (50) for partially accommodating said joining means (23, 24).

11. The apparatus according to claim 10, **characterized in that** each of said joining means (23, 24) comprises two arc-like joining members (37, 38), adjustable connecting members (39) which mutually and adjustably connect said joining members (37, 38), and articulated connecting members (40), which articulately connect said joining members (37, 38) to said articulation means (21, 22).

12. The apparatus according to claim 11, **characterized in that** said adjustable connecting members (39) are provided at first ends (41) of said joining members (37, 38) with two elongated slots (42) formed in said joining members (37, 38), a locking screw (43) inserted in said elongated slots (42), and a fixing nut (44) which is screwed onto said locking screw (43) until said joining members (37, 38) are locked together.

13. The apparatus according to claim 12, **characterized in that** said articulated connecting members (40) are provided at second ends (45) of said joining members (37, 38) and comprise tabs (46) which are connected to said second ends (45) by articulation screws (47) which allow to articulate said tabs (46) with respect to said joining members (37,38).

14. The apparatus according to one or more of the preceding claims, **characterized in that** said locking means (19, 20), said articulation means (21, 22), said joining means (23, 24) and said guiding means (25, 26) are made of radiotransparent materials.

## Patentansprüche

1. Eine Vorrichtung (13) zur externen Fixierung des Beckenrings (14) linke Greifmittel (15) und rechte Greifmittel (16) zum Eingreifen in die Einführ-/Eingriffsbohrungen umfassend, die jeweils am linken (17) und rechten (18) Darmbein des Beckenrings (14) ausgebildet sind, linke Verriegelungsmittel (19) und rechte Verriegelungsmittel (20) zur gegenseitigen Verriegelung der linken Greifmittel (15) bzw. der rechten Greifmittel (16), linke Gelenkmittel (21) und rechte Gelenkmittel (22), die dazu ausgebildet sind, die linken (19) und rechten (20) Verriegelungsmittel gelenkig zu verbinden, und transabdominale Verbindungsmittel (23, 24) zum Verbinden der linken und rechten Gelenkmittel (21, 22); linke und rechte Führungsmittel (25, 26) zum Führen der Bohrmittel des Chirurgen, um die Einführ-/Eingriffsbohrungen vorzunehmen, sodass eine korrekte Ausführung der Einführ-/Eingriffsbohrungen möglich ist; die Vorrichtung ist **dadurch gekennzeichnet, dass** die Führungsmittel (25, 26) Stützkörper (48) umfassen, die ein im Wesentlichen sförmig gebogenes Profil aufweisen.

2. Die Vorrichtung gemäß Anspruch 1, **dadurch gekennzeichnet, dass** das Profil im Wesentlichen die Umfangsform der Darmbeine ungefähr im zentralen Bereich zwischen dem Beckenkamm und der Schamfuge aufweist.

3. Die Vorrichtung gemäß Anspruch 1, **dadurch gekennzeichnet, dass** die linken und rechten Greifmittel (15, 16) Stabelemente umfassen, die über eine Spitze mit einem Außengewinde (27) an einem Ende verfügen, dazu geeignet, durch Drehen zumindest teilweise in die Einführ-/Eingriffsbohrungen eingeführt und in Eingriff gebracht zu werden, sowie über einen eckigen Spitzenteil (28) am gegenüberliegenden Ende, um passend zum Betätigungsmittel zu sein.

4. Die Vorrichtung gemäß Anspruch 3, **dadurch gekennzeichnet, dass** die linken und rechten Verriegelungsmittel (19, 20) in den Führungsmitteln (25, 26) ausgebildete Schlitze (29), Befestigungsmittel (30) zum Befestigen der Greifmittel (15, 16) an den Führungsmitteln (25, 26) und in den Führungsmitteln (25, 26) ausgebildete Führungsbohrungen (31) zum Eingreifen in die Gelenkmittel (21, 22) umfassen.

5. Die Vorrichtung gemäß Anspruch 4, **dadurch gekennzeichnet, dass** die Schlitze (29) in einem länglichen zentralen Bereich der Führungsmittel (25, 26) ausgebildet sind.

6. Die Vorrichtung gemäß Anspruch 4, **dadurch gekennzeichnet, dass** die Führungsbohrungen (31) längs eines zur Außenseite der Vorrichtung (13) gerichteten Umlaufprofils der Führungsmittel (25, 26) ausgebildet sind.

7. Die Vorrichtung gemäß Anspruch 4, **dadurch gekennzeichnet, dass** die Befestigungsmittel (30) Eingriffsschrauben (32) zum Eingreifen in die Greifmittel (15, 16) und Sicherungsmuttern (33) zum Sichern der Eingriffsschrauben (32) umfassen, um die Eingriffsschrauben (32) an den Führungsmitteln (25, 26) zu befestigen.

8. Die Vorrichtung gemäß Anspruch 7, **dadurch gekennzeichnet, dass** jede Eingriffsschraube (32) einen Bolzen umfasst, der an einem Ende über einen Gewindeabschnitt mit Außengewinde passend zur Sicherungsmutter (33) verfügt, und am gegenüberliegenden Ende zum Gewindeabschnitt über einen Kopf, der eine Form passend zu einem Betätigungsmittel aufweist und nahe dem Kopf über eine Querbohrung in dem Bolzen verfügt und von einem der Greifmittel (15, 16) passiert wird.

9. Die Vorrichtung gemäß Anspruch 8, **dadurch gekennzeichnet, dass** die Gelenkmittel (21, 22) Gelenkschrauben (34) zum Eingreifen in die Verbindungsmittel (23, 24), Gelenk-Distanzstücke (35) zum Anbringen an den Gelenkschrauben (34) und an den Gelenkschrauben (34) angebrachte Gelenkmuttern (36) zum Befestigen der Gelenkschrauben (34) an den Führungsmitteln (25, 26) umfassen.

10. Die Vorrichtung gemäß Anspruch 9, **dadurch gekennzeichnet, dass** jede Gelenkschraube (34) einen Gelenkbolzen (51) umfasst, der an einem Ende über einen Abschnitt mit einem Außengewinde passend zur Gelenkmutter (36) verfügt, und am gegenüberliegenden Ende über einen Kopf (53), der eine Form passend zum Betätigungsmittel aufweist und nahe dem Kopf über eine Querbohrung (50) zur teilweisen Aufnahme der Verbindungsmittel (23, 24) verfügt.

11. Die Vorrichtung gemäß Anspruch 10, **dadurch gekennzeichnet, dass** jedes Verbindungsmittel (23, 24) zwei bogenförmige Verbindungsteile (37, 38), einstellbare Verbindungselemente (39), die die Verbindungsteile (37, 38) miteinander und einstellbar verbinden, sowie gelenkige verbindungselemente (40), die die verbindungsteile (37, 38) mit den Gelenkmitteln (21, 22) gelenkig verbinden, umfasst.

12. Die Vorrichtung gemäß Anspruch 11, **dadurch gekennzeichnet, dass** die einstellbaren verbindungselemente (39) an den ersten Enden (41) der Verbindungsteile (37, 38) angebracht sind, wobei zwei Längsschlitze (42) in den Verbindungsteilen (37, 38) ausgebildet sind, eine Befestigungsschraube (43) in den Längsschlitzen (42) eingeführt ist und eine Feststellmitter (44) auf die Befestigungsschraube (43) aufgeschraubt wird bis die Verbindungsteile (37, 38) aneinander befestigt sind.

13. Die Vorrichtung gemäß Anspruch 12, **dadurch gekennzeichnet, dass** die gelenkigen Verbindungselemente (40) an den zweizen Enden (45) der Verbindungsteile (37, 38) angebracht sind und Stifte (46) umfassen, die mittels Gelenkschrauben (47) mit den zweiten Enden (45) verbunden sind, die die gelenkige Anbringung der Stifte (46) in Bezug auf die Verbindungsteile (37, 38) ermöglichen.

14. Die Vorrichtung gemäß einem oder mehreren der vorstehenden Ansprüche, **dadurch gekennzeichnet, dass** die Verriegelungsmittel (19, 20), die Gelenkmittel (21, 22), die Verbindungsmittel (23, 24) und die Führungsmittel (25, 26) aus strahlentransparentem Material bestehen.

## Revendications

1. Appareil (13) pour la fixation externe de l'anneau pelvien (14), comprenant un moyen de préhension gauche (15) et un moyen de préhension droit (16) destinés à être mis en prise dans des trous d'insertion/de mise en prise formés respectivement dans l'os iliaque gauche (17) et droit (18) dudit anneau pelvien (14), un moyen de blocage gauche (19) et un moyen de blocage droit (20) pour mutuellement bloquer respectivement ledit moyen de préhension gauche (15) et ledit moyen de préhension droit (16), un moyen d'articulation gauche (21) et un moyen d'articulation droit (22) adaptés à raccorder de manière articulée lesdits moyens de blocage gauche (19) et droit (20), et des moyens de jonction transabdominaux (23, 24) pour joindre lesdits moyens d'articulation gauche et droit (21, 22) ; des moyens de guidage gauche et droit (25, 26) pour guider un moyen de forage utilisé par le chirurgien pour fournir lesdits trous d'insertion/de mise en prise pour permettre l'exécution correcte desdits trous d'insertion/de mise en prise ; ledit appareil étant **caractérisé en ce que** lesdits moyens de guidage (25, 26) comprennent des corps de support (48) qui ont un profil incurvé sensiblement en forme de S.

2. Appareil selon la revendication 1, **caractérisé en ce que** ledit profil reproduit sensiblement la forme périphérique des os iliaques approximativement dans la région centrale comprise entre la crête iliaque et la symphyse pubienne.

3. Appareil selon la revendication 1, **caractérisé en ce que** lesdits moyens de préhension gauche et droit (15, 16) comprennent des éléments tiges ayant, au niveau d'une extrémité, une pointe dotée d'un filetage externe (27) conçue pour être insérée et mise en prise par vissage au moins partiellement dans lesdits trous d'insertion/de mise en prise et, au niveau de l'extrémité opposée, une partie de pointe de forme carrée (28) destinée à permettre le couplage avec un outil d'actionnement.

4. Appareil selon la revendication 3, **caractérisé en ce que** les moyens de blocage gauche et droit (19, 20) comprennent des fentes (29) formées dans lesdits moyens de guidage (25, 26), des éléments de fixation (30) pour fixer lesdits moyens de préhension (15, 16) auxdits moyens de guidage (25, 26), et des trous de guidage (31) formés dans lesdits moyens de guidage (25, 26) pour la mise en prise desdits moyens d'articulation (21, 22).

5. Appareil selon la revendication 4, **caractérisé en ce que** lesdites fentes (29) sont fournies dans une région centrale longitudinale desdits moyens de guidage (25, 26).

6. Appareil selon la revendication 4, **caractérisé en ce que** lesdits trous de guidage (31) sont formés le long d'un profil périphérique desdits moyens de guidage (25, 26) qui est dirigé vers l'extérieur dudit appareil (13).

7. Appareil selon la revendication 4, **caractérisé en ce que** lesdits moyens de fixation (30) comprennent des vis de mise en prise (32) pour mettre en prise lesdits moyens de préhension (15, 16) et des écrous de blocage (33) pour bloquer lesdites vis de mise en prise (32) de manière à bloquer lesdites vis de mise en prise (32) sur lesdits moyens de guidage (25, 26).

8. Appareil selon la revendication 7, **caractérisé en ce que** chacune desdites vis de mise en prise (32) comprend une tige dotée, au niveau d'une extrémité, d'une partie filetée ayant un filetage externe conçu pour s'accoupler au dit écrou de blocage (33) et, au niveau de l'extrémité opposée relativement à ladite partie filetée, d'une tête ayant une forme adaptée à s'accoupler avec un outil d'actionnement et dotée, à proximité de ladite tête, d'un trou transversal formé dans ladite tige et traversé par l'un desdits moyens de préhension (15, 16).

9. Appareil selon la revendication 8, **caractérisé en ce que** ledit moyen d'articulation (21, 22) comprend une vis d'articulation (34) pour la mise en prise desdits moyens de jonction (23, 24), des écarteurs d'articulation (35) à ajuster sur lesdites vis d'articulation (34), et des écrous d'articulation (36) mise en prise sur lesdites vis d'articulation (34) de manière à bloquer lesdites vis d'articulation (34) sur lesdits moyens de guidage (25, 26).

10. Appareil selon la revendication 9, **caractérisé en ce que** chacune desdites vis d'articulation (34) comprend une tige d'articulation (51) dotée, au niveau d'une extrémité, d'une partie ayant un filetage externe adapté à s'accoupler au dit écrou d'articulation (36) et, au niveau de l'extrémité opposée, d'une tête (53) ayant une forme adaptée à s'accoupler avec un outil d'actionnement et est dotée, à proximité de ladite tête, d'un trou transversal (50) pour partiellement accueillir lesdits moyens de jonction (23, 24).

11. Appareil selon la revendication 10, **caractérisé en ce que** chacun desdits moyens de jonction (23, 24) comprend deux éléments de jonction de type arc (37, 38), des éléments de raccordement ajustables (39) qui raccordent mutuellement et de manière ajustable lesdits éléments de jonction (37, 38) et des éléments de raccordement articulés (40), qui raccordent de manière articulée lesdits éléments de jonction (37, 38) auxdits moyens d'articulation (21, 22).

12. Appareil selon la revendication 11, **caractérisé en ce que** lesdits éléments de raccordement ajustables (39) sont dotés, au niveau de premières extrémités (41) desdits éléments de jonction (37, 38), de deux fentes allongées (42) formées dans lesdits éléments de jonction (37, 38), d'une vis de blocage (43) insérée dans lesdites fentes allongées (42), et d'un écrou de fixation (44) qui est vissé sur ladite vis de blocage (43) jusqu'à ce que lesdits éléments de jonction (37, 38) soient bloqués ensemble.

13. Appareil selon la revendication 12, **caractérisé en ce que** lesdits éléments de raccordement articulés (40) sont fournis au niveau de secondes extrémités (45) desdits éléments de jonction (37, 38) et comprennent des languettes (46) qui sont raccordées auxdites secondes extrémités (45) par des vis d'articulation (47) qui permettent d'articuler lesdites languettes (46) relativement auxdits éléments de jonction (37, 38).

14. Appareil selon l'une ou plusieurs des revendications précédentes, **caractérisé en ce que** lesdits moyens de blocage (19, 20), lesdits moyens d'articulation (21, 22), lesdits moyens de jonction (23, 24) et lesdits moyens de guidage (25, 26) sont faits en matériaux radiotransparents.
